# FASCICULE DE BREVET EUROPEEN

(11) **EP 3 576 639 B1**
(45) Date de publication et mention de la délivrance du brevet: **16.12.2020**
(21) Numéro de dépôt: 18702771.9
(22) Date de dépôt: 18.01.2018
(51) Int. Cl.: A61B 17/06, A61L 17/00, A61L 17/10, A61L 17/12

(54) **FIL CHIRURGICAL BIORÉSORBABLE ET PROCÉDÉ DE FABRICATION D'UN TEL FIL**
BIORESORBIERBARER CHIRURGISCHER DRAHT UND VERFAHREN ZUR HERSTELLUNG SOLCH EINES DRAHTS
BIORESORBABLE SURGICAL WIRE AND METHOD FOR PRODUCING SUCH A WIRE

(30) Priorité: 02.02.2017 FR 1750872
(43) Date de publication de la demande: 11.12.2019
(73) Titulaire: 1st Surgiconcept, 59200 Tourcoing (FR)
(72) Inventeur: POUPONNEAU, Pierre, 25000 Besançon (FR); FAIVRE, Nicolas, 39700 Falletans (FR); PERROT, Sébastien, 25000 Besançon (FR)
(74) Mandataire: Novagraaf Technologies
(86) Numéro de dépôt international: PCT/FR2018/050123
(87) Numéro de publication internationale: WO 2018/142041

(56) Documents cités:
- EP-A1- 2 815 771
- EP-A1- 2 815 771
- US-A- 5 127 413

## Description

La présente invention concerne de manière générale un fil chirurgical biorésorbable de soutien destiné à être notamment utilisé en chirurgie reconstructive, qui présente des propriétés mécaniques améliorées et se dégradent lentement dans le temps.

La chirurgie reconstructive est un domaine en croissance depuis son apparition après la première guerre mondiale. Le marché mondial de cette chirurgie représentait 5,5 milliards d'euros en 2013 dont 1,3 milliards d'euros en Europe et devrait être multiplié par deux d'ici 2019.

La régénération du maintien des tissus, et plus particulièrement le maintien de la peau, constituent 13% des interventions en chirurgie reconstructive. La réalisation de ce type de chirurgie nécessite des dispositifs médicaux, généralement des fils de soutien implantés dans les tissus.

Parmi les dispositifs médicaux (désignés ci-après par l'acronyme DM), on distingue dispositifs médicaux permanents des dispositifs médicaux biorésorbables (désignés ci-après par l'acronyme DMB). Ces derniers se dégradent progressivement, généralement selon un procédé hydrolytique, selon un mois à quatre ans. Cette propriété de dégradation au cours du temps est certes intéressante pour le patient dans le cadre d'une procédure chirurgicale, car l'utilisation d'un DMB n'implique aucune intervention post-opératoire pour l'éliminer du corps du patient, celui-ci s'éliminant progressivement. Mais, les DM biorésorbables présentent de nombreux inconvénients, tels que : le coût élevé de leur matière première (3000 à 6000 €/kg vs. 300 à 1000€/kg pour du silicone médical), la dégradation de leurs propriétés mécaniques lors de leur mise en forme, la rigidité intrinsèque des polymères utilisés, la perte progressive de leurs propriétés mécaniques après implantation dans le corps humain, et enfin un risque non négligeable de réaction inflammatoire.

A l'heure actuelle, environ 200 000 interventions de chirurgie reconstructive sont réalisées par an dans le monde, qui nécessitent chacune environ six fils de soutien. Les fils de soutien comportent typiquement un manchon avec des picots qui permettent de tirer et suspendre les tissus. Le principe d'action de ces fils est de tendre, grâce aux picots, les tissus afin de permettre leur réorganisation, c'est-à-dire la génération de collagène entre les tissus, qui a disparu en raison du vieillissement de la personne. Le remodelage du tissu sera d'autant plus efficace que le fil de soutien pourra garder ses propriétés mécaniques le plus longtemps possible. Ainsi l'effet de la chirurgie reconstructrice pourra être d'autant plus longtemps prolongé. Pour son implantation, le fil doit être à la fois souple (déformation de 10 à 20%) et résistant aux sollicitations mécaniques du chirurgien (8 N).

Actuellement, 80% des fils de soutien sont biorésorbables. On connait notamment quatre types de fils de soutien actuellement disponibles dans le commerce. En particulier, on peut notamment citer les fils de soutien biorésorbables, qui sont commercialisés par la société américaine SINCLAIR sous la marque *«Silhouette Soft* ®» et « Silhouette Lift® ». Ce fil commercial est composé d'un monofilament en acide L-polylactique (PLLA) sur lequel sont surmoulés des cônes acide poly(lactique-co-glycolique) (PLGA). On peut encore citer les fils de soutien commercialisés par la société russe Aptos sous la marque *«Aptos thread*®*»* et par la société italienne PromoItalia sous la marque *«Happy Lift*®*».* Il s'agit dans les deux cas de fils avec picots en acide poly(lactique-co-caprolactone) (ou P(LA-CL)). De par la nature de leurs polymères et la probable diminution des propriétés du matériau lors de leur mise en forme, ces produits sont caractérisés par une dégradation relativement rapide de leurs propriétés mécaniques. Il en résulte que l'effet de la chirurgie reconstructive est limité à 1 an au plus, et oblige ainsi le patient à subir une nouvelle intervention. Les matériaux utilisés dans dispositifs médicaux présentent un temps de dégradation complet de 3 à 6 mois pour le PLGA, 6 à 12 mois pour le PDO, 12 à 18 mois pour le P(LA-CL) et de 1 à 2 ans pour le PLLA. Les fils de suture à base de PDO et P(LA-CL) perdent 50% de leur tenue mécanique à 3 et 6 semaines pour un temps de dégradation complet de 26 semaines. Normalement, le temps de dégradation devrait être de l'ordre d'au moins 52 semaines. La différence est issue de la dégradation de la matière lors de sa transformation. Généralement, le fil est fabriqué par extrusion ce qui nécessite de chauffer assez longuement la matière.

Malgré la perte rapide des propriétés mécaniques après implantation, ces fils de soutien sont quand même intéressants car ils sont souples et permettent leur bonne maniabilité du DM par le chirurgien. *A contrario,* Les matériaux biorésorbables, qui conservent leurs propriétés mécaniques après plusieurs mois d'implantation comme les PLLA de haut poids moléculaire, sont caractérisés par une grande rigidité ce qui est en inadéquation avec le besoin de souplesse nécessaire à l'implantation du fil de soutien. En outre, le besoin de souplesse limite la masse de tissu que peut supporter le fil de soutien. Il est donc nécessaire d'utiliser plusieurs fils pour une opération.

Enfin, on peut encore citer la demande de brevet anglais GB2537033 qui décrit un fil de suture pour la chirurgie esthétique comprenant un filament biorésorbable comportant à titre de picots des éléments tronconiques. Ces fils ne comportent pas de manchon.

Afin de pallier les inconvénients précités, la demanderesse a mis au point un fil de soutien biorésorbable, un fil d'âme constitué d'un filament bobiné moulé dans un manchon souple en polymère biorésorbable entourant le fil d'âme le long duquel sont disposées des picots.

Les fils chirurgicaux en polymère biorésorbables sont connus de l'homme de l'art. Ainsi, la demande de brevet américain US 2007/219587 décrit un fil biorésorbable présentant à sa surface des projections, des épines inclinées, des crochets ou des saillies de formes multiples (assimilables à des picots), qui sont distribués en spirale autour du fil. Selon un mode de réalisation particulier, ce fil chirurgical peut être constitué d'un fil droit avec picots, autour duquel est enroulé un autre fil extérieur pour apporter un effet « ressort ». Les picots sont donc partiellement ou complètement cachés par le ou les fils extérieurs.

Par ailleurs, la demande de brevet européen EP 2687238 décrit un fil de suture bioabsorbable comprenant un fil d'âme en un composant bioabsorbable à bas point de fusion, et une pluralité de fils d'enroulement constitués d'un composant bioabsorbable à température de fusion plus élevée, ces fils d'enroulement étant enroulés autour du fil d'âme. Ce fil de suture a l'inconvénient majeur de ne pas présenter de picots extérieurs, servant à tirer et suspendre les tissus.

EP2815771 décrit une suture chirurgicale comprenant un fil d'âme et un manchon avec des picots qui peuvent être constitués de polymères biorésorbables. La suture peut être revêtue ou imprégnée d'un principe actif.

La présente invention a donc pour objet un fil chirurgical biorésorbable de soutien, caractérisé en ce qu'il comprend :
- un fil d'âme constitué d'un filament bobiné d'un polymère choisi dans le groupe des polyesters et des polyuréthanes biorésorbables, et
- un manchon en polymère biorésorbable entourant le fil d'âme, ce manchon comprenant une section droite le long de laquelle sont disposées des picots (32) inclinés et contenus dans au moins un plan (33).

Le fil chirurgical biorésorbable selon l'invention présente les propriétés suivantes : (*i*) souplesse lors de son maniement, (*ii*) tenue mécanique satisfaisante après 6 mois de dégradation *in vitro,* (*iii*) tenue mécanique satisfaisante pour son implantation.

De manière avantageuse, le polymère constituant le fil d'âme peut être un polyester choisi dans le groupe des acides polylactiques (PLA), des acides glycoliques (PGA), des copolymères desdits acides polylactiques (PLA) et glycoliques (PGA), des copolymères d'ε-caprolactone, des copolymères d'ε-caprolactone et d'acide lactique, et de leurs mélanges.

On utilisera de préférence l'acide L-polylactique (PLLA) en tant que polymère constituant le fil d'âme. Le PLLA est caractérisé par un module d'Young élevé mais une élongation faible. Le temps de dégradation est supérieur à un an^{[1]}.

De manière avantageuse, le polymère constituant le manchon peut être le ε-polycaprolactone ou un copolymère d'ε-caprolactone. Le PCL et ses copolymères est caractérisé par un faible module d'Young mais une grande élongation^{[1]}. De manière avantageuse, on pourra utiliser à titre de polymère constitutif du manchon le copolymère d'ε-polycaprolactone et d'acide lactique rapport molaire compris entre 20/80 et 40/60, et de préférence de l'ordre de 30/70.

De manière avantageuse, le manchon souple en polymère biorésorbable peut en outre comprendre un principe actif et / ou un agent de contraste.

De manière avantageuse, les picots du manchon peuvent être disposés dans un même plan symétriquement de part et d'autre de la section droite du fil manchon, ces picots étant inclinés avec un même angle d'inclinaison α par rapport à une section droite.

De manière avantageuse, le fil chirurgical selon l'invention peut en outre comprendre au moins un picot supplémentaire disposé dans un plan perpendiculaire au plan contenant les autres picots et la section droite.

La présente invention a également pour objet un procédé de fabrication d'un fil chirurgical biorésorbable, caractérisé en ce qu'il comprend les étapes suivantes :
A) moulage du manchon comprenant les sous-étapes suivantes :
   - A1) Fourniture d'un moule présentant une empreinte creuse ayant la forme du manchon ;
   - A2) Introduction à froid d'un polymère biorésorbable sous forme pulvérulente (notamment sous forme de granulés ou en poudre) dans l'empreinte ;
   - A3) Chauffage jusqu'à ce que le polymère biorésorbable soit dans un état au moins pseudo visqueux ;
   - A4) spatulage pour que le polymère biorésorbable remplisse complètement le moule ;
B) Refroidissement du manchon dans son moule pendant 10 à 30 minutes, jusqu'à solidification du polymère biorésorbable et obtention dudit manchon ;
C) Démoulage du manchon.

Le procédé selon l'invention présente l'avantage d'être un procédé de fabrication aisément industrialisable.

Le polymère biorésorbable utilisable dans le cadre du procédé selon l'invention pour réaliser le manchon est tel que défini précédemment. Ainsi, de manière avantageuse, on pourra utiliser à titre de polymère biorésorbable pour réaliser le manchon le ε-polycaprolactone (ou PCL) ou un copolymère d'ε-caprolactone. De préférence, on pourra utiliser à titre de polymère constitutif du manchon le copolymère d'ε-polycaprolactone et d'acide lactique avec rapport molaire compris entre 20/80 et 40/60, et de préférence de l'ordre de 30/70.

De manière avantageuse, on peut lors de l'étape A3) du procédé selon l'invention, introduire en outre, dans l'empreinte, un principe actif et / ou un agent de contraste.

De manière avantageuse, on peut utiliser un moule souple, ce qui permet d'éviter l'utilisation de démoulant.

De manière avantageuse, on peut immerger (étape B1) un fil d'âme dans le moule tant que le manchon est à l'état pseudo-visqueux.

De manière avantageuse lors de l'étape A2, on ajoute dans l'empreinte, en même temps que le polymère biorésorbable sous forme pulvérulente, un principe actif et/ou un agent de contraste.

De manière avantageuse, l'agent de contraste ou le principe actif peuvent être mélangés au polymère biorésorbable, à l'aide ou non d'un dispersant, avant le dépôt dans l'empreinte.

A titre de principes actifs utilisables dans le cadre de la présente invention, on peut notamment citer des agents antibactériens ou antalgiques. Afin d'améliorer le mélange du principe actif ou agent de contraste, un agent dispersant peut aussi être ajouté.

Le polymère utilisable dans le cadre du procédé selon l'invention à titre de fil d'âme est également tel que défini précédemment.

Ainsi, de manière avantageuse, le polymère constituant le fil d'âme peut être un polyester choisi dans le groupe des acides polylactiques (PLA), des acides glycoliques (PGA), des copolymères desdits acides polylactiques (PLA) et glycoliques (PGA), des copolymères d'ε-caprolactone, des copolymères d'ε-caprolactone et d'acide lactique, et de leurs mélanges. On utilisera de préférence l'acide L-polylactique (PLLA) en tant que polymère constituant le fil d'âme.

De préférence, le procédé selon l'invention peut en outre comprendre les étapes suivantes :
- préalablement à l'étape A) de moulage du manchon, une étape A0) de préparation d'un fil d'âme par bobinage d'un filament autour d'un mandrin ;
- après le démoulage C) du manchon intégrant le fil d'âme autour du mandrin (4), une étape de retrait D) du mandrin.

Le mandrin permet de faciliter le positionnement du fil d'âme dans le manchon.

D'autres caractéristiques et avantages de la présente invention apparaîtront plus clairement à la lecture de la description suivante donnée à titre d'exemple illustratif et non limitatif et faite référence aux figures annexées dans lesquelles :
- La figure 1 est une photographie représentant un fil chirurgical constitué uniquement d'un manchon en P(LA-CL) ;
- La figure 2 regroupe trois photographies (figures 2a, 2b et 2c) d'un fil d'âme pour un fil chirurgical selon l'invention, ce fil d'âme étant constitué d'un filament bobiné sur un mandrin, pour différents bobinages sur le mandrin ;
- La figure 3 est une photographie représentant un fil chirurgical résorbable selon l'invention ;
- La figure 4 montre le moulage du mandrin sur lequel un filament en PLLA a été bobiné, dans un manchon en PCL ;
- Les figures 5a et 5b montrent le fil d'âme surmoulé dans le manchon en PCL de la figure 4, obtenu après retrait du mandrin ;
- Les figures 6a et 6b montrent le moulage d'un fil d'âme en PLLA sans mandrin dans un manchon en PCL ;
- Les figures 7a et 7b sont deux photographies représentant un fil chirurgical selon l'invention avec un fil d'âme en PLLA et un manchon en PCL, comportant des picots disposés dans deux plans perpendiculaires ;
- La figure 8 montre l'évolution au cours du temps d'un fil de soutien selon l'invention, lors d'un test de vieillissement ;
- La figure 9 montre l'évolution de la force de rupture mesurée lors d'essais mécaniques de traction réalisés pour un fil de soutien selon l'invention, un manchon sans fil d'âme, et le fil d'âme lui-même.

Dans les figures, on utilise les mêmes références numériques pour désigner les mêmes éléments.

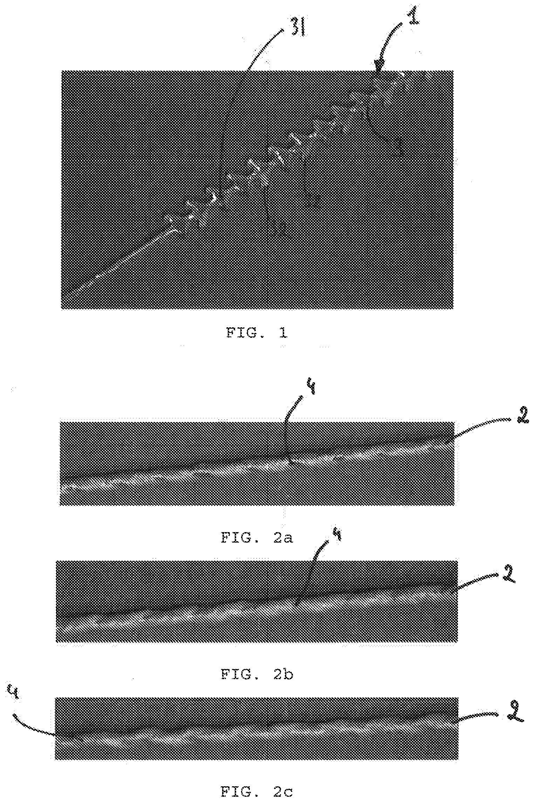

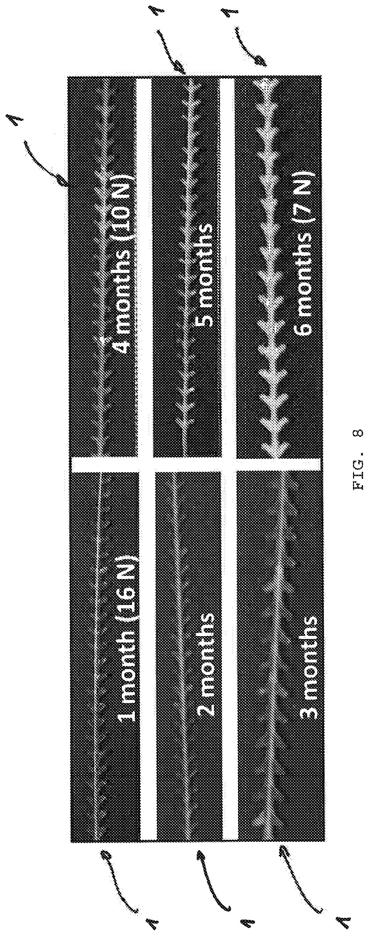

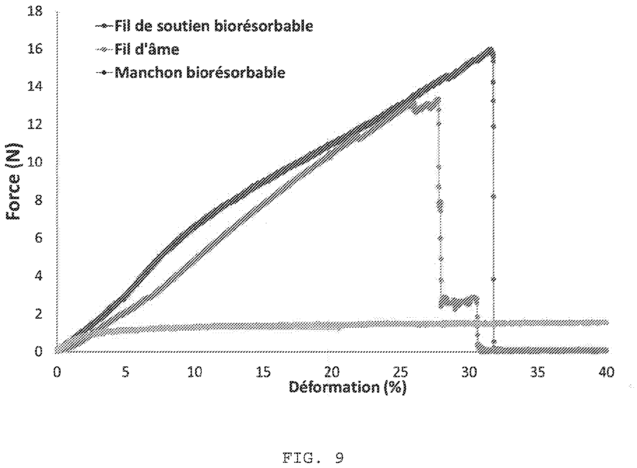

La figure 1 est une photographie représentant un fil 1 chirurgical constitué uniquement d'un manchon en acide poly(lactique-caprolactone) (ou P(LA-CL)) : les tests réalisés dans les exemples ci-après (cf. également les figures 8 et 9) montrent qu'un tel fil chirurgical se dégrade plus vite dans le temps qu'un fil chirurgical selon l'invention. Le fil illustré sur la figure 1 se différencie d'un fil chirurgical selon l'invention par l'absence de fil d'âme 3 surmoulé dans le manchon 3.

Un tel fil d'âme 2 est illustré (non moulé dans un manchon) par les photographies des figures 2a, 2b et 2c : le fil d'âme 2 est constitué d'un filament bobiné (conformément à l'étape A0 du procédé selon l'invention) sur un mandrin, pour différents bobinages sur le mandrin. Sur la figure 2a, le diamètre du mandrin est de 0,3 mm, le fil a été bobiné avec un pas de 1,5 avec 4 passages ; sur la figure 2b, le diamètre du mandrin est de 0,3 mm également, le fil a été bobiné avec un pas de 1,5 avec 6 passages ; sur la figure 2c, le diamètre du mandrin est de 0,2 mm, le fil a été bobiné avec un pas de 1,5 avec 0,6 passages. Le bobinage permet d'augmenter la force à la rupture du fil d'âme 2.

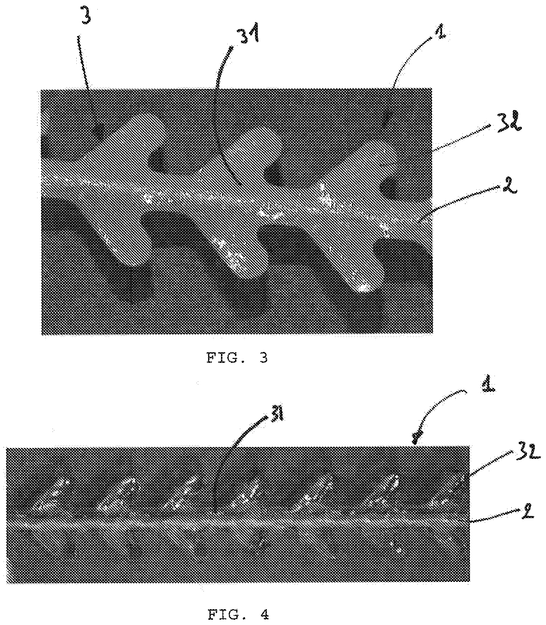

La figure 3 montre un fil chirurgical résorbable selon l'invention comprenant en fil d'âme en PLLA et un manchon en PCL. Cette figure montre que le fil d'âme est parfaitement centré au milieu du manchon. Les tests réalisés dans les exemples ci-après (cf. également les figures 8 et 9) montrent qu'un tel fil chirurgical présentent des propriétés améliorées en termes de vieillissement et de résistance à la traction par rapport aux fils représentés sur les figures 1 et 2 (correspondant respectivement au manchon seul et au fil d'âme seul). Le fil sans le manchon se dégrade (mécaniquement) plus vite qu'avec le manchon.

La figure 4 montre le moulage du mandrin sur lequel un filament en PLLA a été bobiné (conformément à l'étape B1), dans un manchon en PCL.

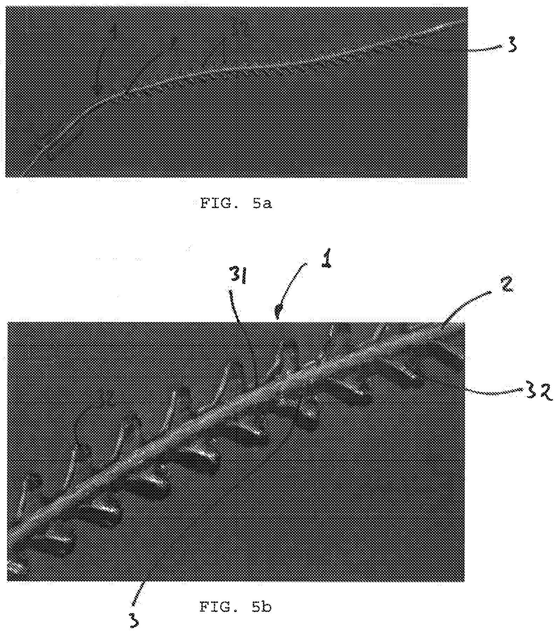

Après retrait du mandrin (conformément à l'étape D du procédé selon l'invention), on obtient le fil de soutien biorésorbable selon l'invention représenté sur les figures 5a et 5b. Le surmoulage du mandrin autour duquel est enroulé le fil d'âme facilite la pénétration dans le manchon.

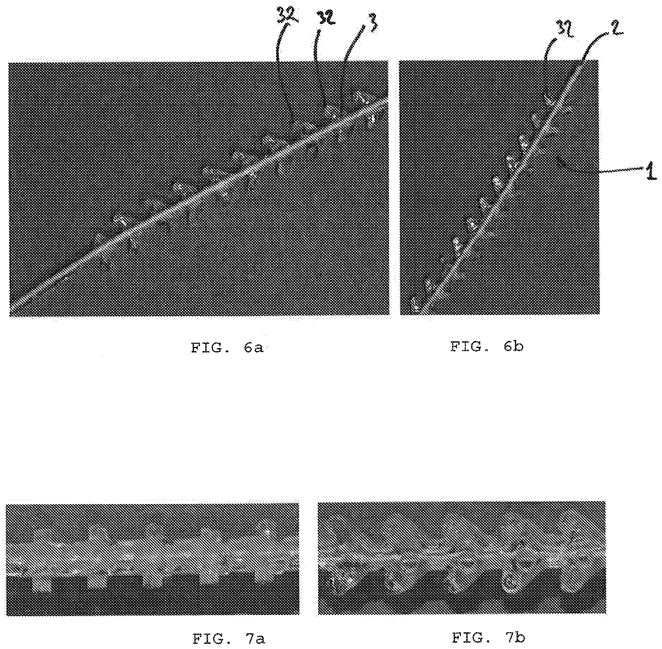

Il est possible de placer, lors de l'étape B1 du procédé selon l'invention, le fil d'âme dans le manchon sans passer par une étape préalable de préparation par enroulement d'un filament 21 autour d'un mandrin 4. En effet, les figures 6a et 6b montrent le moulage d'un fil d'âme en PLLA sans mandrin dans un manchon en PCL. Toutefois, le placement du fil d'âme dans le manchon est un peu plus délicat et compliqué qu'avec le mandrin.

Les figures 7a et 7b sont deux photographies représentant un fil chirurgical selon l'invention avec un fil d'âme en PLLA et un manchon en acide poly(lactique-caprolactone) (ou P(LA-LC)), comportant des picots disposés dans deux plans perpendiculaires. Pour obtenir cette variante de fil de soutien selon l'invention, deux manchons ont été surmoulés autour du fil d'âme. Pour cela, chaque moule est pourvu d'un guidage. On introduit le matériau polymère constitutif du manchon dans les deux moules, puis on spatule. On introduit alors le fil d'âme dans le premier moule en cours de refroidissement, et ensuite on ajoute le deuxième moule. Puis, on comprime à l'aide d'une presse les deux moules.

Les figures 8 et 9 sont commentées dans les exemples qui suivent, servant à comparer les propriétés mécaniques et de vieillissement de fils de soutien selon l'invention avec des fils commerciaux selon l'art antérieur.

Dans ces exemples, sauf indication contraire, tous les pourcentages et parties sont exprimés en pourcentages massiques.

### EXEMPLES

### Produits

- Fil 1 biorésorbable selon l'invention : il est constitué d'un fil de renfort tressé à partir d'un fil d'âme commercial en PLLA multibrins associé à un manchon réalisé avec de l'acide poly(lactique-co-ε-caprolactone) ou du poly(ε-caprolactone).
- Fil constitué uniquement d'un manchon 3 en P(LA-CL) (ratio 70/30) ;
- Fil d'âme 2 bobiné en acide PLLA ou en P(LA-CL).

### Tests de caractérisation mécanique

### Test de vieillissement

Il s'agit d'essais de dégradation de fils de soutien selon l'invention uniquement.

Ces fils ont été placés dans une solution tamponnées de phosphate de sodium à 50°C (commercialisée sous la dénomination commerciale PBS AMRESCO, 0780-10L, lot : 1405C066) sous agitation (200 rpm). Pour ce test, en raison de la température, 1 semaine d'immersion correspond à 1 mois (37°C).

La solution a été renouvelée après 3 semaines. Le volume a été maintenu à 7L par ajout ponctuel de PBS. Afin d'étudier le phénomène de fluage de ces fils, les fils selon l'invention sont également soumis à une force de 1 N : une masse (92 ± 1 g) a été fixé à l'extrémité des fils placés dans la solution de PBS.

### Essais mécaniques de traction :

Les essais mécaniques ont été réalisés avec une machine de traction (Chatillon : TCD200) équipée d'un système d'acquisition (Andilog : Center Touch).

### EXEMPLE 1 : résultats des tests de dégradation sur le fil de soutien selon l'invention

Dans cet essai, une semaine correspond à un mois *in vivo.* (37°C). La figure 8 montre que :
- Jusqu'à 5 mois, aucune variation significative de la masse du fil de soutien n'a été constatée. Ce résultat confirme que la dégradation de la matière n'a pas été accélérée par sa transformation.
- A 6 mois, la perte de masse est de 16 ± 3 %. La dégradation est visible par le changement d'aspect du fil de soutien. A 6 mois, à la rupture, la force est de 7 ± 0.3 N et la déformation est de 9 ± 3 %.
- Ainsi, à 24 semaines, le fil n'a perdu que 50% de ses propriétés mécaniques et sa force de rupture reste 3 fois supérieure à sa charge en fonctionnement.

### EXEMPLE 2 : résultats des essais comparatifs de traction

La figure 9 montre les résultats des essais de traction pour le fil biorésorbable selon l'invention, que l'on compare à ceux de son manchon 3 seul d'une part, et à ceux réalisés sur le fil d'âme 2 d'autre part.

Le fil d'âme 2 en PLLA confère au DM sa résistance mécanique. Afin d'optimiser ses propriétés, le fil est tressé. Les paramètres du tressage (pas, nombre de passage) influencent les propriétés résultantes. Ainsi, grâce à cette conception, les propriétés mécaniques du fil 1 ont été optimisées (le manchon en P(LA-CL) ayant de faibles propriétés mécaniques par rapport au fil d'âme) . La force à la rupture du fil tressé atteint 14 N alors que celle d'un fil non tressé est de 4 N. Le procédé de mise en forme du fil, qui ne nécessite aucune source de chaleur, ne dégrade pas les propriétés initiales du fil. Le bobinage du fil peut être optimisé selon la masse de tissus à soutenir.

Le manchon 3 apporte au fil de soutien (ou DM) les picots nécessaires au maintien des tissus. Le manchon est moulé à une température très contrôlée, légèrement au-dessus de la température de fusion, afin de ne pas dégrader la matière et donc ses propriétés. Il est réalisé à partir d'un copolymère biorésorbable commercial qui présente un temps de dégradation complet entre 12 et 18 mois. Ce matériau présente un comportement viscoélastique, caractérisé par un allongement important.

### Liste des références

[1] Biomaterials 21 (2000) 2335-2346.

## Revendications

1. Fil chirurgical biorésorbable de soutien (1), **caractérisé en ce qu'**il comprend :
- un fil d'âme (2) constitué d'un filament bobiné d'un polymère choisi dans le groupe des polyesters et des polyuréthanes biorésorbables, et
- un manchon (3) souple en polymère biorésorbable entourant le fil d'âme (2), ledit manchon (3) comprenant une section droite (31) le long de laquelle sont disposées des picots (32).

2. Fil selon la revendication 1, selon lequel le polymère constituant le fil d'âme (2) est un polyester choisi dans le groupe des acides polylactiques (PLA), des acides glycoliques (PGA), des copolymères desdits acides polylactiques (PLA) et glycoliques (PGA), des copolymères d'ε-caprolactone, des copolymères d'ε-caprolactone et d'acide lactique, et de leurs mélanges, et de préférence l'acide L-polylactique (PLLA).

3. Fil selon l'une quelconque des revendications 1 à 2 selon lequel polymère biorésorbable constituant le manchon (3) est le ε-polycaprolactone ou un copolymère d'ε-caprolactone.

4. Fil selon la revendication 3, selon lequel le manchon est constitué d'un copolymère d's-polycaprolactone et d'acide lactique avec un rapport molaire compris entre 20/80 et 40/60, et de préférence de l'ordre de 30/70.

5. Fil selon la revendication 1, selon lequel le manchon (3) souple en polymère biorésorbable peut en outre comprendre un principe actif et / ou un agent de contraste.

6. Fil selon l'une quelconque des revendications 1 à 5, dans lequel les picots (32) du manchon (3) sont disposés dans un même plan (33) symétriquement de part et d'autre de la section droite (31) du fil manchon (3), lesdits picots (32) étant inclinés avec un même angle d'inclinaison α par rapport à ladite section droite (31).

7. Fil selon la revendication 6, comprenant en outre au moins un picot (32) supplémentaire disposé dans un plan perpendiculaire audit plan (33).

8. Procédé de fabrication d'un fil chirurgical biorésorbable tel que défini selon l'une quelconque des revendications 1 à 3, **caractérisé en ce qu'**il comprend les étapes suivantes :
A) Moulage du manchon (3) comprenant les sous-étapes suivantes :
• A1) Fourniture d'un moule présentant une empreinte creuse ayant la forme du manchon (2) ;
• A2) Introduction à froid d'un premier polymère sous forme pulvérulente dans l'empreinte ;
• A3) Chauffage jusqu'à ce premier polymère soit dans un état au moins pseudo visqueux ;
• A4) spatulage pour que ledit premier polymère remplisse complètement le moule ;
B) Refroidissement du manchon (3) dans son moule pendant 10 à 30 minutes, jusqu'à solidification dudit premier polymère et obtention du manchon (3) ;
C) Démoulage du manchon (3).

9. Procédé selon la revendication 8, dans lequel le moule (5) est un moule souple.

10. Procédé selon la revendication 8 ou 9, selon lequel polymère biorésorbable constituant le manchon (3) est le ε-polycaprolactone ou un copolymère d'ε-caprolactone, et de préférence un copolymère d'ε-polycaprolactone et d'acide lactique avec un rapport molaire compris entre 20/80 et 40/60, et de préférence de l'ordre de 30/70.

11. Procédé selon l'une quelconque des revendications 8 à 10, dans lequel lors de l'étape A3), on introduit en outre, dans l'empreinte, un principe actif et / ou un agent de contraste.

12. Procédé selon l'une quelconque des revendications 8 à 11, dans lequel on immerge B1), un fil d'âme (2) dans ledit moule (5) tant que le manchon (3) est à l'état pseudo-visqueux.

13. Procédé selon la revendication 12, dans lequel le polymère constituant le fil d'âme (2) est un polyester choisi dans le groupe des acides polylactiques (PLA), des acides glycoliques (PGA), des copolymères desdits acides polylactiques (PLA) et glycoliques (PGA), des copolymères d'ε-caprolactone, des copolymères d'ε-caprolactone et d'acide lactique, et de leurs mélanges, et de préférence est l'acide L-polylactique (PLLA).

14. Procédé selon l'une quelconque des revendications 8 à 13, comprenant en outre les étapes suivantes :
- préalablement à l'étape A) de moulage du manchon (3), une étape A0) de préparation du fil d'âme (2) par bobinage d'un filament (21) autour d'un mandrin (4) ;
- après le démoulage C) du manchon (3) intégrant le fil d'âme (2) autour du mandrin (4), retrait D) dudit mandrin (4).

15. Procédé selon l'une quelconque des revendications 8 à 14, dans lequel le polymère constituant le fil d'âme (2) est un polyester choisi dans le groupe des acides polylactiques (PLA), des acides glycoliques (PGA), des copolymères desdits acides polylactiques (PLA) et glycoliques (PGA), des copolymères d'ε-caprolactone, des copolymères d'ε-caprolactone et d'acide lactique, et de leurs mélanges, et de préférence est l'acide L-polylactique (PLLA).

## Patentansprüche

1. Bioresorbierbarer chirurgischer Stützfaden (1), **dadurch gekennzeichnet, dass** er umfasst:
- einen Kernfaden (2), der aus einem gewickelten Filament aus einem Polymer besteht, das aus der Gruppe von bioresorbierbaren Polyestern und Polyurethanen ausgewählt ist, und
- eine flexible Hülse (3) aus bioresorbierbarem Polymer, die den Kernfaden (2) umgibt, wobei die Hülse (3) einen geraden Abschnitt (31) umfasst, entlang dem Zacken (32) angeordnet sind.

2. Faden nach Anspruch 1, wobei das Polymer, das den Kernfaden (2) bildet, ein Polyester ist, der aus der Gruppe von Polymilchsäuren (PLA), Glykolsäuren (PGA), Copolymeren von den Polymilchsäuren (PLA) und Glykolsäuren (PGA), Copolymeren von ε-Caprolacton, Copolymeren von ε-Caprolacton und Milchsäure und deren Gemischen und vorzugsweise L-Polymilchsäure (PLLA) ausgewählt ist.

3. Faden nach einem der Ansprüche 1 bis 2, wobei das bioresorbierbare Polymer, das die Hülse (3) bildet, ε-Caprolacton oder ein Copolymer von ε-Caprolacton ist.

4. Faden nach Anspruch 3, wobei die Hülse aus einem Copolymer von ε-Caprolacton und Milchsäure mit einem Molverhältnis besteht, das zwischen 20:80 und 40:60 und vorzugsweise in der Größenordnung von 30:70 liegt.

5. Faden nach Anspruch 1, wobei die flexible Hülse (3) aus bioresorbierbarem Polymer weiterhin einen Wirkstoff und/oder ein Kontrastmittel umfassen kann.

6. Faden nach einem der Ansprüche 1 bis 5, wobei die Zacken (32) der Hülse (3) in derselben Ebene (33) symmetrisch auf beiden Seiten des geraden Abschnitts (31) der Fadenhülse (3) angeordnet sind, wobei die Zacken (32) mit demselben Neigungswinkel α in Bezug auf den geraden Abschnitt (31) geneigt sind.

7. Faden nach Anspruch 6, weiterhin umfassend mindestens einen zusätzlichen Zacken (32), der in einer Ebene angeordnet ist, die senkrecht zu der Ebene (33) ist.

8. Verfahren zur Herstellung eines bioresorbierbaren chirurgischen Fadens wie nach einem der Ansprüche 1 bis 3 definiert, **dadurch gekennzeichnet, dass** es die folgenden Schritte umfasst:
A) Gießen der Hülse (3), umfassend die folgenden Unterschritte:
• A1) Bereitstellen einer Gießform, aufweisend eine hohle Vertiefung mit der Form der Hülse (2);
• A2) kaltes Einbringen eines ersten Polymers in Pulverform in die Vertiefung;
• A3) Erhitzen, bis dieses erste Polymer in einem zumindest pseudoviskosen Zustand ist;
• A4) Spachteln, damit das erste Polymer die Gießform vollständig füllt;
B) Abkühlen der Hülse (3) in ihrer Gießform für 10 bis 30 Minuten bis zur Verfestigung des ersten Polymers und zum Erhalt der Hülse (3);
C) Entformen der Hülse (3).

9. Verfahren nach Anspruch 8, wobei die Gießform (5) eine flexible Gießform ist.

10. Verfahren nach Anspruch 8 oder 9, wobei das bioresorbierbare Polymer, das die Hülse (3) bildet, ε-Caprolacton oder ein Copolymer von ε-Caprolacton und vorzugsweise ein Copolymer von ε-Caprolacton und Milchsäure mit einem Molverhältnis ist, das zwischen 20:80 und 40:60 und vorzugsweise in der Größenordnung von 30:70 liegt.

11. Verfahren nach einem der Ansprüche 8 bis 10, wobei während dem Schritt A3) weiterhin ein Wirkstoff und/oder ein Kontrastmittel in die Gießform eingebracht wird.

12. Verfahren nach einem der Ansprüche 8 bis 11, wobei B1) ein Kernfaden (2) in die Gießform (5) eingetaucht wird, während die Hülse (3) in dem pseudoviskosen Zustand ist.

13. Verfahren nach Anspruch 12, wobei das Polymer, das den Kernfaden (2) bildet, ein Polyester ist, der aus der Gruppe von Polymilchsäuren (PLA), Glykolsäuren (PGA), Copolymeren von den Polymilchsäuren (PLA) und Glykolsäuren (PGA), Copolymeren von ε-Caprolacton, Copolymeren von ε-Caprolacton und Milchsäure und deren Gemischen ausgewählt ist und vorzugsweise L-Polymilchsäure (PLLA) ist.

14. Verfahren nach einem der Ansprüche 8 bis 13, weiterhin umfassend die folgenden Schritte:
- vor dem Schritt A) des Gießens der Hülse (3) einen Schritt A0) des Herstellens des Kernfadens (2) durch Wickeln eines Filaments (21) um einen Dorn (4);
- nach dem Entformen C) der Hülse (3), die den Kernfaden (2) um den Dorn (4) integriert, Herausziehen D) des Dorns (4).

15. Verfahren nach einem der Ansprüche 8 bis 14, wobei das Polymer, das den Kernfaden (2) bildet, ein Polyester ist, der aus der Gruppe von Polymilchsäuren (PLA), Glykolsäuren (PGA), Copolymeren von den Polymilchsäuren (PLA) und Glykolsäuren (PGA), Copolymeren von ε-Caprolacton, Copolymeren von ε-Caprolacton und Milchsäure und deren Gemischen ausgewählt ist und vorzugsweise L-Polymilchsäure (PLLA) ist.

## Claims

1. Bioresorbable surgical support wire (1), **characterised in that** it comprises:
- a core wire (2) consisting of a wound filament of a polymer selected from the group of bioresorbable polyesters and polyurethanes, and
- a flexible sleeve (3) made from bioresorbable polymer surrounding the core wire (2), said sleeve (3) comprising a cross-section (31) along which picots (32) are arranged.

2. Wire according to claim 1, whereby the polymer forming the core wire (2) is a polyester selected from the group of polylactic acids (PLA), glycolic acids (PGA), copolymers of said polylactic (PLA) and glycolic (PGA) acids, copolymers of ε-caprolactone, copolymers of ε-caprolactone and lactic acid, and mixtures thereof, and preferably poly-L-lactic acid (PLLA).

3. Wire according to any one of claims 1 to 2, whereby the bioresorbable polymer forming the sleeve (3) is ε-caprolactone or a copolymer of ε-caprolactone.

4. Wire according to claim 3, whereby the sleeve consists of a copolymer of ε-caprolactone and lactic acid with a molar ratio between 20:80 and 40:60, and preferably of the order of 30:70.

5. Wire according to claim 1, whereby the flexible bioresorbable polymer sleeve (3) can further comprise an active substance and/or a contrast agent.

6. Wire according to any one of claims 1 to 5, wherein the picots (32) of the sleeve (3) are disposed in the same plane (33) symmetrically on either side of the cross-section (31) of the sleeve wire (3), said picots (32) being inclined with the same angle of inclination α with respect to said cross-section (31).

7. Wire according to claim 6, further comprising at least one additional picot (32) disposed in a plane perpendicular to said plane (33).

8. Method for manufacturing a bioresorbable surgical wire as defined according to any one of claims 1 to 3, **characterised in that** it comprises the following steps:
A) Moulding the sleeve (3) comprising the following sub-steps:
• A1) Supplying a mould having a hollow cavity having the shape of the sleeve (2);
• A2) Cold insertion of a first polymer in powder form into the cavity;
• A3) Heating until this first polymer is in an at least pseudo-viscous state;
• A4) Spatulating so that said first polymer fills the mould completely;
B) Cooling the sleeve (3) in the mould thereof for 10 to 30 minutes, until the first polymer has solidified and the sleeve (3) is obtained;
C) Releasing the sleeve (3) from the mould.

9. Method according to claim 8, wherein the mould (5) is a flexible mould.

10. Method according to claim 8 or 9, whereby the bioresorbable polymer forming the sleeve (3) is ε-polycaprolactone or a copolymer of ε-polycaprolactone, and preferably a copolymer of ε-polycaprolactone and lactic acid with a molar ratio between 20:80 and 40:60, and preferably of the order of 30:70.

11. Method according to any one of claims 8 to 10, wherein during step A3), an active substance and/or a contrast agent is furthermore introduced into the cavity.

12. Method according to any one of claims 8 to 11, wherein B1) a core wire (2) is submerged in said mould (5) while the sleeve (3) is in the pseudo-viscous state.

13. Method according to claim 12, wherein the polymer forming the core wire (2) is a polyester selected from the group of polylactic acids (PLA), glycolic acids (PGA), copolymers of said polylactic (PLA) and glycolic (PGA) acids, copolymers of ε-caprolactone, copolymers of ε-caprolactone and lactic acid, and mixtures thereof, and preferably is poly-L-lactic acid (PLLA).

14. Method according to any one of claims 8 to 13, further comprising the following steps:
- prior to step A) of moulding the sleeve (3), a step A0) of preparing the core wire (2) by winding a filament (21) around a mandrel (4);
- after releasing from the mould C) the sleeve (3) incorporating the core wire (2) around the mandrel (4), removing D) said mandrel (4).

15. Method according to any one of claims 8 to 14, wherein the polymer forming the core wire (2) is a polyester selected from the group of polylactic acids (PLA), glycolic acids (PGA), copolymers of said polylactic (PLA) and glycolic (PGA) acids, copolymers of ε-caprolactone, copolymers of ε-caprolactone and lactic acid, and mixtures thereof, and preferably is poly-L-lactic acid (PLLA).
